# EUROPÄISCHE PATENTSCHRIFT

(11) **EP 2 692 385 B1**
(45) Veröffentlichungstag und Bekanntmachung des Hinweises auf die Patenterteilung: **28.02.2018**
(21) Anmeldenummer: 13003047.1
(22) Anmeldetag: 14.06.2013
(51) Int. Cl.: A61M 16/16

(54) **Vorrichtung zur aktiven Atemluftanfeuchtung**
Device for actively moistening breathing air
Dispositif d'humidification active de l'air de respiration

(30) Priorität: 01.08.2012 DE 202012007387 U
(43) Veröffentlichungstag der Anmeldung: 05.02.2014
(73) Patentinhaber: Müller, Gerhard, 23829 Wittenborn (DE)
(72) Erfinder: Müller, Gerhard, 23829 Wittenborn (DE)
(74) Vertreter: Marschall, Stefan

(56) Entgegenhaltungen:
- WO-A1-2011/122964
- US-A1- 2010 258 114

## Beschreibung

Die Neuerung bezieht sich auf eine Vorrichtung zur aktiven Atemluftanfeuchtung bei einer Beatmung von Patienten über eine Maske oder einem Tubus bzw. eine Trachialkanüle, bestehend aus einem Verlängerungselement, zum Durchtritt der Atemluft mit einer außenliegenden Atemöffnung, wobei das Verlängerungselement zweiteilig aus einem oberen Teil für die Atemluft und einem unteren Teil als Anschlusselement mit einer zwischengeschalteten Mischkammer ausgebildet ist.

Ein großer Anteil von tracheotomierten Patienten ist wegen der fehlenden Umgebung des klimatisierenden Mund- und Rachenraumes darauf angewiesen, die Atemluft künstlich mit technischer Unterstützung zu erwärmen und befeuchtet zu bekommen. Dieses wird bei gesunden Menschen von den Schleimhäuten in Mund und Nase übernommen.

Es ist bekannt, diesen Mangel an Wärme und Feuchte überwiegend durch spezielle Klimatisierungsfilter, sogenannte "feuchte Nasen" zu reduzieren. Das Wirkprinzip dieser Filter ist es mithilfe eines beschichteten Trägermediums die in der Ausatemluft enthaltene Feuchtigkeit und Wärme kurzfristig zu speichern und beim nächsten Atemzug die kalte und trockene Frischluft aus dem gespeicherte Feuchte- und Wärmereservoir soweit als physikalisch möglich zu klimatisieren. Es hat sich aber gezeigt, dass durch diese Hilfsmittel die auftretenden Defizite verringert werden, aber physiologisch akzeptable Werte in der Regel sind nicht zu erreichen.

Um die Befeuchtungssituation zu verbessern, wird bei auftretenden Schleimhautproblemen oder einer Verschlechterung der Sekretsituation häufig mehrmals am Tag oder andauernd mit Hilfe eines Ultraschallverneblers angewärmtes Aerosol in die Nähe der Tracheostomaöffnung geblasen. Dieses Aerosol soll dann mit der Atemluft angesaugt werden und zumindest zeitweise die Befeuchtungssituation verbessern

Eine gattungsgemäße Anordnung ist bereits nach der WO2011/122964A1 bekannt geworden. Hierbei besteht der Mangel, dass kein konstanter Querschnitt für die Atemluftzufuhr geschaffen wird, so dass eine Reduzierung des Beatmungswiderstandes erfolgt. Zusätzlich ist nachteilig, dass keine Mischkammer zur Zuführung für eine Befeuchtung besteht, die ohne Verwirbelung eine Zuführung über einen gesonderten Zuführspalt ohne Beeinträchtigung und Verwirbelung der Atemluftzufuhr erfolgt.

Die Aufgabe der Neuerung ist es, eine einfache Vorrichtung zu schaffen, die Schleimhäute funktionsfähig zu erhalten und einen geringen Bedienungsaufwand zu ermöglichen.

Die Lösung dieser Aufgabe erfolgt neuerungsgemäß dadurch, dass der obere Teil des Verlängerungselementes in die Mischkammer zur Bildung eines Ringraumes hineinragt und im Abstand zu einem mit der Mischkammer verbundenen unteren Teil des Verlängerungselementes als Anschlusselement angeordnet ist, und ein Zuführspalt aus der Mischkammer durch das obere Teil des Verlängerungselementes gebildet ist.

In der Zeichnung ist ein Ausführungsbeispiel der Neuerung schematisch dargestellt. Es zeigen:

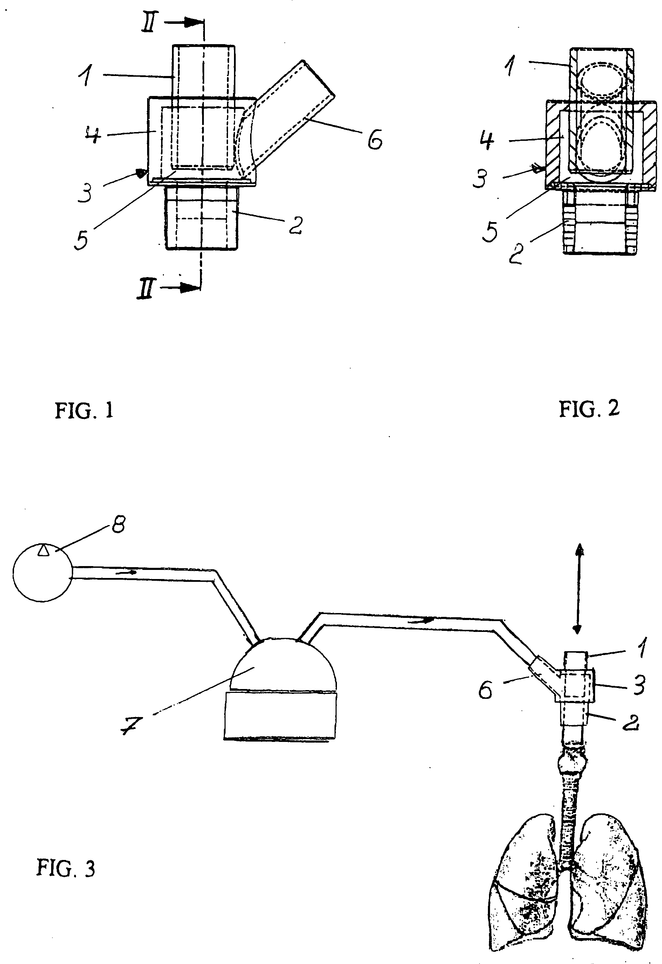

- Fig. 1: eine Vorrichtung mit ihren Anschlüssen,
- Fig. 2: eine Schnittdarstellung der Vorrichtung nach Linie II-II der Fig. 1 und
- Fig. 3: eine eingesetzte Vorrichtung als Funktionsschema

Die Vorrichtung wird im wesentlichen durch ein einsetzbares Verlängerungselement 1, 2 zum Durchtritt der Atemluft gebildet, das durch ein oberes Teil 1 und ein unteres Teil 2 gebildet ist, die im Abstand angeordnet sind.

Das obere Teil 1 des Verlängerungselementes ist dabei in eine zwischengeschaltete Mischkammer 3 eingesetzt, so dass ein Ringraum 4 gebildet wird und das untere Teil 2 des Verlängerungselementes ist mit dem Boden der Mischkammer 3 als Anschlusselement verbunden. Dabei wird durch den gebildeten Abstand zwischen oberem und unterem Teil 1, 2 des Verlängerungselementes ein Zuführspalt 5 aus dem Ringraum 4 gebildet.

In den Ringraum 4 der Mischkammer 3 mündet eine Zuführung 6, die mit einem Atemluftbefeuchter 7 verbunden ist. Der Atemluftbefeuchter 7 ist mit einer regelbaren Druckquelle 8 verbunden, wobei der Atemluftbefeuchter 7 ein steuerbares Heizelement aufweist. Hierdurch ist es möglich, eine Zumischung von erwärmter, befeuchteter Luft zur Spontanatmung bei tracheotomierten Patienten durchzuführen. Mit einem regelbaren Druck wird über die seitliche Zuführung 6 erwärmte, befeuchtete Luft in die Mischkammer 3 geführt und von dort mit der Spontanatmung vom Patienten eingeatmet. Der Vorteil liegt darin, dass die Befeuchtungsleistung wesentlich verbessert wird, ohne dass ein durch Fehlbedienung oder Falschanschluss entstehender Verschluss des Atemweges entsteht.

## Patentansprüche

1. Vorrichtung zur aktiven Atemluftanfeuchtung bei einer Beatmung von Patienten über eine Maske oder einem Tubus bzw. eine Trachialkanüle, bestehend aus einem Verlängerungselement (1, 2), zum Durchtritt der Atemluft mit einer außenliegenden Atemöffnung, wobei das Verlängerungselement (1, 2) zweiteilig aus einem oberen Teil (1) für die Atemluft und einem unteren Teil (2) als Anschlusselement mit einer zwischengeschalteten Mischkammer (3) ausgebildet ist wobei die Mischkammer (3) eine mit dem Atemluftbefeuchter (7) verbundene Zuführung (6) aufweist, die in einem spitzen Winkel zum oberen Teil (1) des Verlängerungselementes angeordnet ist, sowie der Atemluftbefeuchter (7) mit einer regelbaren Druckquelle (8) verbunden ist und ein steuerbares Heizelement aufweist, **dadurch gekennzeichnet, dass** der obere Teil (1) des Verlängerungselementes in die Mischkammer (3) zur Bildung eines Ringraumes (4) hineinragt und im Abstand zu einem mit der Mischkammer (3) verbundenen unteren Teil (2) des Verlängerungselementes als Anschlusselement aufweist und ein Zuführspalt (5) aus der Mischkammer (3) durch das obere Teil (1) des Verlängerungselementes gebildet ist.

## Claims

1. Device for active moistening of the respiratory air in respiration of patients by means of a mask or a tube and/or a tracheal cannula, consisting of an extension element (1, 2) for passage of the respiratory air through an exterior respiratory opening, wherein the extension element (1, 2) is designed in two parts, comprising an upper part (1) for the respiratory air and a lower part (2) as the connecting element with a mixing chamber (3) connected in between, wherein the mixing chamber (3) has a feed (6) connected to the respirator air humidifier (7), which is disposed at an acute angle to the upper part (1) of the extension element, and also the respiratory air humidifier (7) is connected to a regulable pressure source (8) and has a controllable heating element, **characterized in that**
the upper part (1) of the extension element protrudes into the mixing chamber (3) to form an annular space (4) and has as a connecting element at a distance from a lower part (2) of the extension element connected to the mixing chamber (3), and an inlet gap (5) is formed from the mixing chamber (3) is formed by the upper part (1) of the extension element.

## Revendications

1. Dispositif destiné à l'humidification active d'air respiratoire lors d'une ventilation artificielle de patients par le biais d'un masque ou d'un tube, respectivement d'une canule trachéale, composé d'un élément de prolongation (1, 2), pour faire passer l'air respiratoire avec une ouverture de respiration extérieure, dans lequel l'élément de prolongation (1, 2), est formé en deux parties avec une partie supérieure (1) pour l'air respiratoire et une partie inférieure (2) en tant qu'élément de raccordement avec une chambre de mélange (3) montée entre, dans lequel la chambre de mélange (3) présente une alimentation (6) reliée à l'humidificateur d'air respiratoire (7) qui est agencée en angle aigu par rapport à la partie supérieure (1) de l'élément de prolongation, de même que l'humidificateur d'air respiratoire (7) est relié à une source de pression (8) pouvant être réglée et présente un élément de chauffage pouvant être commandé,
**caractérisé en ce que** la partie supérieure (1) de l'élément de prolongation fait saillie dans la chambre de mélange (3) pour former un espace annulaire (4) et présente à distance d'un élément inférieur (2) de l'élément de prolongation relié la chambre de mélange (3) en tant qu'élément de raccordement, et une fente d'alimentation (5) hors de la chambre de mélange (3) est formée par la partie supérieure (1) de l'élément de prolongation.
